Europäisches Patentamt

European Patent Office

Office européen des brevets

(19)

(11) Veröffentlichungsnummer: **0 131 636**
**A1**

(12) # EUROPÄISCHE PATENTANMELDUNG

(21) Anmeldenummer: 83106719.4

(22) Anmeldetag: 08.07.83

(51) Int. Cl.⁴: **A 61 N 1/16**

(43) Veröffentlichungstag der Anmeldung:
23.01.85 Patentblatt 85/4

(84) Benannte Vertragsstaaten:
AT BE CH DE FR GB IT LI LU NL SE

(71) Anmelder: Esper, Herbert, Dipl.-Ing. (FH)
Bürgermeister-Heinrich-Strasse 23
D-8403 Bad Abbach(DE)

(72) Erfinder: Esper, Herbert, Dipl.-Ing. (FH)
Bürgermeister-Heinrich-Strasse 23
D-8403 Bad Abbach(DE)

(74) Vertreter: Graf, Helmut, Dipl.-Ing. et al,
Greflinger Strasse 7 Postfach 382
D-8400 Regensburg(DE)

(54) Gerät zur Abschirmung von Erdstrahlen.

(57) Die Erfindung ist gekennzeichnet durch einen stabförmigen Körper (1) aus Eisen, durch wenigstens zwei spulenartige Wicklungen aus Kupferdraht auf diesem Körper, wobei sich die einzelnen Windungen der einen Wicklung (2', 2'') zwischen den beiden Stirnseiten des stabförmigen Körpers in Längsrichtung dieses Körpers erstrecken und die einzelnen Windungen der anderen Wicklung (3) in Umfangerichtung des stabförmigen Körpers verlaufen, so daß die beiden Wicklungen aus Kupferdraht einen Spulenkäfig bilden.

Fig.3

EP 0 131 636 A1

## Gerät zur Abschirmung von Erdstrahlen

Die Erfindung bezieht sich auf ein Gerät zur Abschirmung insbesondere von ortsfesten Objekten, wie beispielsweise von Häusern, Wohnungen usw., aber auch zum Abschirmen von beweglichen Objekten, wie beispielsweise Kraftfahrzeugen gegenüber Erdstrahlen.

"Erdstrahlen" im Sinne der Erfindung sind Erströme oder Ladungsträgerverschiebungen, die vor allem auch außerhalb der Erdoberfläche bis in relativ große Höhen wirksam sind und bei ständigem Einwirken auf einen menschlichen Körper bzw. Organismus zu erheblichen Störungen (Nervosität, Schlafstörungen usw.), im Exstremfall sogar zu schweren gesundheitlichen Störungen führen können.

Diese Erdströme sind hinsichtlich ihrer Entstehung hauptsächlich auf Vorgänge innerhalb der Erde zurückzuführen. Wie allgemein bekannt sind, besitzt die Erde ein Magnetfeld, welches grundsätzlich dem Magnetfeld eines Stabmagneten entspricht, und zwar mit den Polen im Bereich des Nord- und Südpols der Erde. Überall im Erdboden befindet sich Wasser. Wo dieses Wasser in Bewegung ist, schneidet es die Magnetlinien der Erde. Dabei werden Elektronen verschoben, was zur Entstehung eines elektrischen Stromes führt. Diese Tatsache wurde bereits von dem Physiker Faraday entdeckt.

Weiterhin gibt es in der Erde nahezu überall beieinanderliegende unterschiedliche Materialien, wobei das diese Materialien umgebende, chemisch nicht reine Wasser einen Elektrolyten bildet, so daß hierdurch auch Batterie- bzw. elektrolytische Ströme entstehen können.

Ferner können Ladungsträgerverschiebungen und damit Ströme im Inneren des Erdbodens durch das in Bewegung befindliche Grundwasser entstehen.

0131636

Die so im Inneren der Erde entstehenden Ströme bzw.
Ladungsträger fließen vermutlich durch die Fliehkraft der
Erde zur Erdoberfläche, wobei diese Ströme zwar sehr klein
sind, dennoch bei ihrem Austritt aus der Erdoberfläche zu
den obengenannten Störungen bzw. gesundheitlichen Beeinträchtigungen führen können. Durch die die Erde umgebenden
Magnetlinien des natürlichen Magnetfeldes der Erde aber
auch durch auf menschliche Einflußnahme zurückzuführende
Magnetfelder wird der aus der Erdoberfläche austretende
Erdstrom (Ladungsträger) in einer bestimmten Richtung
gerichtet.

Weiterhin ist davon auszugehen, daß durch Flüssigkeitsbewegungen im menschlichen Körper (beispielsweise durch den
Blutkreislauf) bei Vorhandensein von magnetischen Feldern
Ladungsträgerverschiebungen und damit Ströme entstehen.

Es wurden bereits zahlreiche Versuche unternommen, um den
Lebensraum, beispielsweise Häuser, Wohnungen, Büros usw.,
in dem sich kranke oder auf Erdstrahlen besonders ansprechende, sensible Personen bevorzugt aufhalten, gegen
diese Erdstrahlen abzuschirmen. Diese bekannten Methoden
und Verfahren sind jedoch teilweise nur bedingt wirksam,
oder nur mit großem Aufwand möglich, oder aber setzen, wie
beispielsweise der Versuch einer Abschirmung durch Permanent-Magneten eine sehr exakte Anordnung und Ausrichtung
der zur Abschirmung verwendeten Elemente beispielsweise am
Boden eines Wohn- oder Schlafraumes voraus, und zwar
jeweils direkt dort, wo besondere Störzonen vorliegen, was
vor allem auch bedeutet, daß eine Vielzahl derartiger
Permanent-Magnete unter Umständen in einem Raum am Boden
verteilt angeordnet werden müssen, wodurch die Bewegungsfreiheit in diesem Raum stark beeinträchtigt wird. Weiterhin hat diese Methode auch den Nachteil, daß Permanent-
Magnete auf Dauer ihre Wirkung verlieren und daher eine wirksame Abschirmung nur dann garantiert ist, wenn die einzelnen Permanent-Magnete in regelmäßigen Abständen durch neue Magnete ersetzt oder aber die einzelnen Magnet-Dipole eines solchen Magneten durch eine Eisenbrücke wieder ausgerichtet werden.

Der Erfindung liegt die Aufgabe zugrunde, ein Gerät zur Abschirmung gegen Erdstrahlen aufzuzeigen, welches die Nachteile vorhandener Geräte oder Methoden vermeidet, einfach herstellbar und im Bereich des abzuschirmenden Raumes anbringbar ist sowie über eine beliebig lange Zeit voll wirksam bleibt.

Zur Lösung dieser Aufgabe ist ein Gerät der erfindungsgemäßen Art gekennzeichnet durch einen stabförmigen Körper aus Eisen, durch wenigstens zwei spulenartige Wicklungen aus Kupferdraht auf diesem Körper, wobei sich die einzelnen Windungen der einen Wicklung zwischen den beiden Stirnseiten des stabförmigen Körpers in Längsrichtung dieses Körpers erstrecken und die einzelnen Windungen der anderen Wicklung in Umfangsrichtung des stabförmigen Körpers verlaufen, so daß die beiden Wicklungen aus Kupferdraht eine Art "Spulenkäfig" bilden.

Der Erfindung liegt dabei die Erkenntnis zugrunde, daß durch diamagnetische Stoffe und dabei bevorzugt durch Kupfer der eingangs erwähnte, störend oder sogar schädigend auf den menschlichen Körper bzw. Organismus einwirkende Stromkreislauf zur Erde unterbrochen werden kann, was nach einer weiteren, der Erfindung zugrundeliegenden Erkenntnis auf folgende Fakten zurückzuführen ist:

Bei einem diamagnetischen Werkstoff ist zwischen dem Atomkern und den diesen Atomkern umgebenden Elektronen die Anziehungskraft ausgeglichen. Diamagnetische Stoffe haben die Eigenschaft, vorhandene Magnetfelder zu verdrängen bzw.

eine Art "Anti-Magnetfeld" zu erzeugen, wenn sie in ein Magnetfeld gebracht werden. Entscheidende Faktoren sind dabei Form, Lage und Menge des Werkstoffs, bevorzugt Kupfer. Legt man z.B. Kupfer in ein stromdurchflossenes Magnetfeld, so werden der Stromfluß sowie die Magnetlinien der Erde durch den Aufbau des Anti-Magnetfeldes unterbrochen. Je nach Stärke des Stromflusses sind der Wirkungsbereich oder die Abschirmung enger oder weiter gezogen.

Schließlich liegt der Erfindung die Erkenntnis zugrunde, daß die für eine wirksame Abschirmung erforderliche Kupfermenge dann reduziert werden kann, wenn das Kupfer als Spule über einen Eisenkern gewickelt ist, da in der Spule Strom zur Erzeugung eines Magnetfeldes verbraucht wird, das sich im Eisenkern konzentriert bzw. das den Eisenkern anzieht.

Weitere, bevorzugte Ausbildungen des erfindungsgemäßen Gerätes sind Gegenstand der Unteransprüche.

Die Erfindung wird im folgenden anhand der Figuren, die die einzelnen Phasen der Herstellung einer Ausführungsform des erfindungsgemäßen Gerätes wiedergeben, näher erläutert. Es zeigen:

Fig. 1 in perspektivischer Darstellung ein stabförmiger Körper aus Eisen zur Verwendung bei der Herstellung des erfindungsgemäßen Gerätes;

Fig. 2 in perspektivischer Darstellung den stabförmigen Körper gemäß Fig. 1 zusammen mit einer ersten, auf diesem Körper aufgebrachten Wicklung aus Kupferdraht;

Fig. 3 in perspektivischer Darstellung den stabförmigen Körper nach Fig. 1 und 2 zusammen mit einer zweiten Wicklung aus Kupferdraht, die auf die erste Wicklung aufgebracht wurde;

Fig. 4 einen Längsschnitt durch das fertiggestellte Gerät
gemäß der Erfindung.

In den Figuren ist 1 ein hohlzylinderartiges Rohrstück aus
Eisen, welches bei der dargestellten Ausführungsform den
stabförmigen Körper aus Eisen im Sinne der Erfindung
bildet.

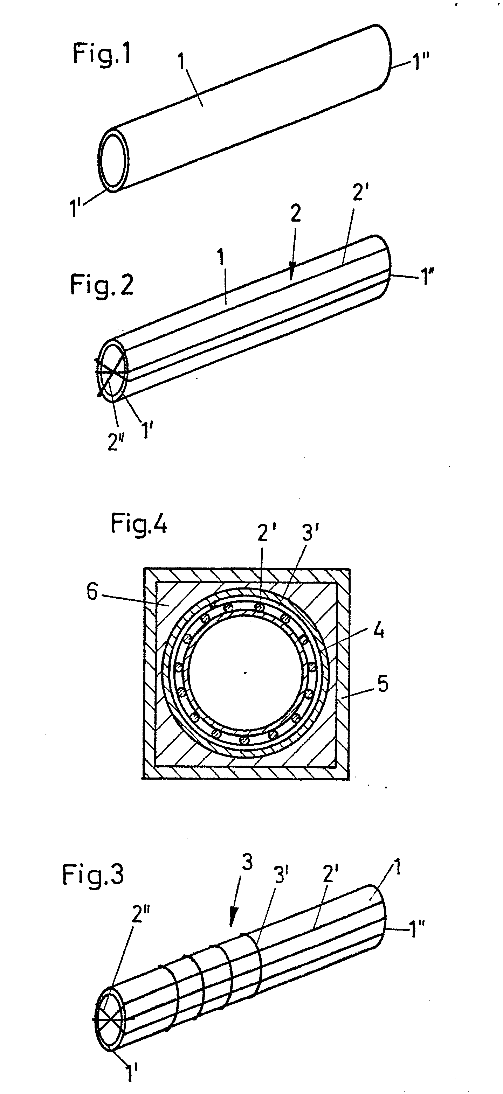

Auf dieses Rohrstück 1 wird entsprechend der Fig. 2
zunächst eine erste Wicklung aus Kupferdraht derart
aufgebracht, daß sich die einzelnen Windungen 2 dieser
Wicklung jeweils über die beiden Stirnseiten bzw. Enden 1'
und 1'' des Rohrstückes 1 erstrecken, d.h. jede Windung 2
besitzt zwei Abschnitte 2', die sich an der Umfangsfläche
des Rohrstückes 1 in Rohrstücklängsrichtung erstrecken und
zwei Abschnitte 2'', die sich diagonal bzw. quer über die
beiden Stirnseiten 1' des Rohrstückes 1 erstrecken, wobei
die Abschnitte 2'' an den beiden Stirnseiten 1' und 1''
sämtlicher Windungen der Wicklung 2 in ihrer Gesamtheit
eine in etwa sternartige Struktur ergeben. Nach dem
Aufbringen der in sich geschlossenen Wicklung 2 aus
Kupferdraht wird auf diese eine zweite Wicklung 3 ebenfalls
aus Kupferdraht aufgebracht, wobei die einzelnen Windungen
3' dieser ebenfalls in sich geschlossenen Wicklung in
Umfangsrichtung des Rohrstückes 1 verlaufen und somit die
Abschnitte 2' der Wicklung 2 im wesentlichen unter einem
Winkel von 90° kreuzen, wodurch sich ein das Rohrstück 1
umschließender Spulenkäfig ergibt.

Das mit den beiden Wicklungen 2 und 3 versehene Rohrstück 1
wird dann in einem weiteren Verfahrensschritt mit einer
Aluminiumfolie 4 umschlossen und anschließend zum Schutz
gegen äußere mechanische Einflüsse in einen Hohlkörper
eingebracht, der von einem Vierkant-Hohlprofil 5, bevorzugt
von einer Vierkant-Hohlprofil aus Eisen gebildet ist. Der
in diesem Hohlprofil 5 verbleibende Raum, insbesondere zwischen der Außenfläche der Aluminiumfolie 4 und der Innenfläche des Hohlprofils 5 sowie an den beiden Enden des Hohlprofiles 5, die über die beiden Stirnseiten des mit den Wicklungen 2 und 3 versehenen Rohrstückes 1 vorstehen, wird mit einer Ausgußmasse 6, z.B. mit Kunststoff, Wachs oder mit einem anderen Material ausgegossen, welches einen dichten Abschluß garantiert und insbesondere auch eine Korrosion der Kupferdrähte der Wicklungen 2 und 3 verhindert.

Es hat sich gezeigt, daß sich in der Regel bereits mit ein bis vier Geräten der beschriebenen Art ein 100 $m^2$ großes Haus wirksam abschirmen läßt. Nur in Ausnahmefällen sind weitere Geräte erforderlich. Bevorzugt können diese Geräte im Kellerboden eingelassen werden, so daß sie nicht unbeabsichtigt entfernt oder aber durch unbeabsichtigtes Gegenstoßes aus ihrer Lage gebracht werden können.

Weiterhin hat sich gezeigt, daß das erfindungsgemäße Gerät auch zum Abschirmen von beweglichen Objekten, wie beispielsweise Personenkraftwagen geeignet ist. In diesem Fall wird beispielsweise ein erfindungsgemäßes Gerät mittig und mit der Längsachse des Rohrstückes 1 in Fahrtrichtung des Kraftfahrzeugs ausgerichtet unter dem Fahrersitz angeordnet.

<u>P a t e n t a n s p r ü c h e</u>

0131636

1. Gerät zur Abschirmung gegenüber Erdstrahlen, gekennzeichnet durch einen stabförmigen Körper (1) aus Eisen, durch wenigstens zwei spulenartige Wicklungen (2, 3) aus Kupferdraht auf diesem Körper (1), wobei sich die einzelnen Windungen (2´, 2´´) der einen Wicklung (2) zwischen den beiden Stirnseiten (1´, 1´´) des stabförmigen Körpers in Längsrichtung dieses Körpers (1) erstrecken und die einzelnen Windungen (3´) der anderen Wicklung (3) in Umfangsrichtung des stabförmigen Körpers (1) verlaufen, so daß die beiden Wicklungen (2, 3) aus Kupferdraht einen Spulenkäfig bilden.

2. Gerät nach Anspruch 1, dadurch gekennzeichnet, daß der stabförmige Körper (1) einen kreisförmigen oder quadratischen oder rechteckförmigen Außenquerschnitt aufweist.

3. Gerät nach Anspruch 1 oder 2, dadurch gekennzeichnet, daß der stabförmige Körper (1) ein Hohlkörper, beispielsweise ein Rohrstück ist.

4. Gerät nach einem der Ansprüche 1 bis 3, gekennzeichnet durch eine den Spulenkäfig umgebende Schicht aus Aluminiumfolie (4).

5. Gerät nach einem der Ansprüche 1 bis 4, gekennzeichnet durch einen, den stabförmigen Körper (1) und den Spulenkäfig sowie ggf. auch die Schicht aus Aluminiumfolie (4) umschließenden weiteren Hohlkörper (5) vorzugsweise aus Eisen.

6. Gerät nach Anspruch 5, dadurch gekennzeichnet, daß der vom stabförmigen Körper (1) und vom Spulenkäfig sowie ggf. von der Schicht aus Aluminiumfolie (4) nicht eingenommene Raum des weiteren Hohlkörpers durch eine Ausgußmasse (6) ausgefüllt ist.

Fig.1

Fig.2

Fig.4

Fig.3

0131636

1/1

**0131636**
Nummer der Anmeldung

Europäisches
Patentamt

**EUROPÄISCHER RECHERCHENBERICHT**

EP  83 10 6719

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | FR-A- 859 878 (VOILLAUME)<br>* Seite 1, Zeile 50 - Seite 2, Zeile 8; Seite 2, Zeilen 42-50 * | 1,2,4 | A 61 N    1/16 |
| Y | FR-A-1 231 915 (VALENTAK)<br>* Seite 1, rechte Spalte * | 1,5 | |
| A | FR-A-1 167 001 (VINCON)<br>* Seite 1, Abschnitt 3,5, rechte Spalte, Abschnitt 1 * | 1-3,5 | |
| A | GB-A-1 579 037 (KING)<br>* Seite 2, Zeile 125 - Seite 3, Zeile 33 * | 1 | |
| A | DE-A-1 937 768 (WIDMANN)<br>* Anspruch 1 * | 1 | RECHERCHIERTE SACHGEBIETE (Int. Cl. 3) |
| A | DE-A- 841 779 (MATZKE)<br>* Seite 2, Zeilen 100-108 * | 4 | A 61 N |
| A | FR-A- 766 899 (MERMET)<br>* Seite 1, Zeilen 17-31 * | 5 | |
| A | GB-A- 664 478 (CHRISTEN)<br>* Seite 1, Zeilen 57-73 * | 6 | |

Der vorliegende Recherchenbericht wurde für alle Patentansprüche erstellt.

| Recherchenort<br>DEN HAAG | Abschlußdatum der Recherche<br>02-03-1984 | Prüfer<br>SIMON J.J.E. |
|---|---|---|